# EUROPEAN PATENT APPLICATION

(11) **EP 4 098 755 A1**
(43) Date of publication of application: **07.12.2022**
(21) Application number: 21747758.7
(22) Date of filing: 28.01.2021
(51) Int. Cl.: C12Q 1/6886

(54) **COMPOSITION USING CPG METHYLATION CHANGES IN SPECIFIC GENES TO DIAGNOSE BLADDER CANCER, AND USE THEREOF**

(30) Priority: 28.01.2020 KR 20200010053
(71) Applicant: Gencurix Inc., Seoul 08394 (KR)
(72) Inventor: CHO, Sang Rae, Seoul 08394 (KR); MOON, Young Ho, Seoul 08394 (KR); HAN, Jinil, Seoul 08394 (KR)
(74) Representative: Richardt Patentanwälte PartG mbB
(86) International application number: PCT/KR2021/001154
(87) International publication number: WO 2021/154009

(57) **Abstract**

The present invention relates to a composition, a kit, a nucleic acid, and a method capable of diagnosing bladder cancer by detecting the methylation level of a CpG site of at least one gene selected from the group consisting of IFFO1, MARCH11, BARHL2, NR2E1, KCNA3, and BOLL. According to the present invention, since the hypermethylation of a CpG site of a gene is specifically exhibited in bladder cancer, a composition, a kit, a chip, or a method according to the present invention can be used to accurately and rapidly diagnose bladder cancer and to be used for early diagnosis and monitoring recurrence.

## Description

### TECHNICAL FIELD

This application claims the priority of Korean Patent Application No. 10-2020-0010053, filed on January 28, 2020, the entirety of which is a reference of the present application.

The present invention relates to a composition, a kit, a nucleic acid, and a method capable of diagnosing bladder cancer by detecting the methylation level of a CpG site of at least one gene selected from the group consisting of IFFO1, MARCH11, BARHL2, NR2E1, KCNA3, and BOLL.

### BACKGROUND ART

Bladder cancer is a cancer that occurs in the bladder mucosa and is the most common cancer among urological cancers. The most common symptom of bladder cancer is hematuria, and a ratio of bladder cancer as a cause of hematuria is about 25% of the elderly over 65 years of age. In general, when the symptoms of hematuria or bladder irritation persist, a possibility of bladder cancer is suspected and a test for diagnosing bladder cancer is performed.

The most basic test for bladder cancer is urinalysis and cytology. The urinalysis is a test for the presence of red blood cells and inflammatory cells in the urine, and the cytology is a test of determining whether cancer cells have fallen out of the urine. If these tests are positive, there is a high probability to have cancers in the urinary system, including bladder cancer. In addition, there is an advantage of being a non-invasive test and low in price, but there is a disadvantage of low accuracy.

Cystoscopy is performed when the bladder cancer is suspected through the urinalysis or cytology and hematuria is visually observed. In this test, while an endoscope is inserted into the urethra under local anesthesia, the presence, location, shape, number, and size of tumors in the bladder are confirmed by directly observing the presence of cancer in the bladder. However, this method has disadvantages in that the test cost is expensive and it is invasive and it is difficult to be detected in the case of early bladder cancer.

Early detection of bladder cancer is linked to a good prognosis of cancer and is directly related to the quality of life. In addition, since bladder cancer recurs frequently and periodic follow-up examinations are essential, it is necessary to overcome the disadvantages of various existing bladder cancer diagnosis methods and to develop a non-invasive test method that efficiently enables early diagnosis and recurrence monitoring.

Meanwhile, epigenetics is a field that studies the regulation of gene expression in a state in which the base sequence of DNA is not changed. The epigenetics is to study the regulation of gene expression through epigenetic variations, such as DNA methylation, acetylation, methylation, phosphorylation, and ubiquitination of miRNAs or histones, and the like.

Among them, the DNA methylation is the most studied epigenetic variation. The epigenetic variation may result in gene function variations and changes to tumor cells. Accordingly, the DNA methylation is associated with the expression (or inhibition and induction) of regulatory genes of intracellular diseases, and recently, cancer diagnosis methods through measurement of DNA methylation have been proposed. In particular, since cancer-specific methylation occurs in advance even in precancerous tissues, the detection of cancer-specific methylation is highly likely to be used for diagnosis of cancer.

Therefore, it is necessary to develop effective bladder cancer-specific methylation markers capable of predicting the risk of bladder cancer.

### DISCLOSURE

### TECHNICAL PROBLEM

Therefore, the present inventors found that CpG sites in specific genes were hypermethylated in bladder cancer, developed a composition, a kit, a nucleic acid chip, and a method capable of diagnosing bladder cancer by detecting the methylation levels, and then completed the present invention.

An object of the present invention is to provide a composition for diagnosing bladder cancer comprising an agent for measuring the methylation level of a CpG site of a specific gene.

An object of the present invention is also to provide a composition for diagnosing bladder cancer consisting of an agent for measuring the methylation level of a CpG site of a specific gene.

An object of the present invention is also to provide a composition for diagnosing bladder cancer essentially consisting of an agent for measuring the methylation level of a CpG site of a specific gene.

Another object of the present invention is to provide a kit for diagnosing bladder cancer comprising a PRC primer pair for amplifying fragments including a CpG site of a specific gene and a sequencing primer for pyrosequencing a PCR product amplified by the primer pair.

Yet another object of the present invention is to provide a nucleic acid chip for diagnosing bladder cancer in which a probe capable of hybridizing with fragments including a CpG site of a specific gene under a stringent condition are immobilized.

Still another object of the present invention is to provide a method for providing information for diagnosing bladder cancer comprising measuring and comparing the methylation levels of CpG sites of specific genes from different samples.

Still yet another object of the present invention is to provide a method for measuring the methylation level of a CpG site of a specific gene in a biological sample isolated from a subject in order to provide information required for diagnosing the recurrence or progression of bladder cancer.

Still yet object of the present invention is to provide the use of an agent for measuring the methylation level of a CpG site of at least one gene selected from the group consisting of IFFO1, MARCH11, BARHL2, NR2E1, KCNA3 and BOLL for preparing an agent for diagnosing bladder cancer.

Still yet another object of the present invention is to provide a method for diagnosing bladder cancer comprising the steps of:
a) obtaining a sample from a subject;
b) measuring the methylation level of a CpG site of at least one gene selected from the group consisting of IFFO1, MARCH11, BARHL2, NR2E1, KCNA3 and BOLL from the sample; and
c) comparing the measured methylation level with the methylation level of a CpG site of the same gene in a normal control sample.

### TECHNICAL SOLUTION

In order to achieve the object, the present invention provides a composition for diagnosing bladder cancer comprising an agent for measuring the methylation level of a CpG site of at least one gene selected from the group consisting of IFFO1, MARCH11, BARHL2, NR2E1, KCNA3 and BOLL.

In addition, the present invention provides a composition for diagnosing bladder cancer consisting of an agent for measuring the methylation level of a CpG site of at least one gene selected from the group consisting of IFFO1, MARCH11, BARHL2, NR2E1, KCNA3 and BOLL.

In addition, the present invention provides a composition for diagnosing bladder cancer essentially consisting of an agent for measuring the methylation level of a CpG site of at least one gene selected from the group consisting of IFFO1, MARCH11, BARHL2, NR2E1, KCNA3 and BOLL.

In order to achieve another object, the present invention provides a kit for diagnosing bladder cancer comprising a primer pair for amplifying fragments including a CpG site of at least one gene selected from the group consisting of IFFO1, MARCH11, BARHL2, NR2E1, KCNA3 and BOLL.

In order to achieve yet another object, the present invention provides a nucleic acid chip for diagnosing bladder cancer in which a probe capable of hybridizing with fragments including a CpG site of at least one gene selected from the group consisting of IFFO1, MARCH11, BARHL2, NR2E1, KCNA3 and BOLL is immobilized.

In order to achieve still another object, the present invention provides a method for providing information for diagnosing bladder cancer comprising measuring the methylation level of a CpG site of at least one gene selected from the group consisting of IFFO1, MARCH11, BARHL2, NR2E1, KCNA3 and BOLL from a sample of a patient suspected of having bladder cancer; and
comparing the measured methylation level with the methylation level of a CpG site of the same gene in a normal control sample.

In order to achieve still yet another object, the present invention provides a method for measuring the methylation level of a CpG site of at least one gene selected from the group consisting of IFFO1, MARCH11, BARHL2, NR2E1, KCNA3 and BOLL in a biological sample isolated from a subject, in order to provide information required for diagnosing the recurrence or progression of bladder cancer.

In order to achieve still yet another object, the present invention provides the use of an agent for measuring the methylation level of a CpG site of at least one gene selected from the group consisting of IFFO1, MARCH11, BARHL2, NR2E1, KCNA3 and BOLL for preparing an agent for diagnosing bladder cancer for preparing an agent for diagnosing bladder cancer.

In order to achieve still yet another object, the present invention provides a method for diagnosing bladder cancer comprising the steps of:
a) obtaining a sample from a subject;
b) measuring the methylation level of a CpG site of at least one gene selected from the group consisting of IFFO1, MARCH11, BARHL2, NR2E1, KCNA3 and BOLL from the sample; and
c) comparing the measured methylation level with the methylation level of a CpG site of the same gene in a normal control sample.

Unless otherwise defined, all technical and scientific terms used herein have the same meaning as commonly understood by those skilled in the art. The following references provide one skill with general definitions of various terms used in the present specification: Singleton et al., DICTIONARY OF MICROBIOLOGY AND MOLECULAR BIOLOTY(2th ed. 1994); THE CAMBRIDGE DICTIONARY OF SCIENCE AND TECHNOLOGY(Walkered., 1988); and Hale & Marham, THE HARPER COLLINS DICTIONARY OF BIOLOGY.

Hereinafter, the present invention will be described in detail.

The present invention provides a composition for diagnosing bladder cancer comprising an agent for measuring the methylation level of a CpG site of at least one gene selected from the group consisting of IFFO1, MARCH11, BARHL2, NR2E1, KCNA3 and BOLL.

As used herein, the term "methylation" means that a methyl group is attached to bases constituting DNA. Preferably, in the present invention, the methylation refers to whether methylation occurs in cytosine of a specific CpG site of a specific gene. When the methylation occurs, the binding of a transcription factor is disturbed to inhibit the expression of the specific gene. Conversely, when unmethylation or hypermethylation occurs, the expression of the specific gene is increased.

In genomic DNA of mammalian cells, in addition to A, C, G, and T, there is the fifth base called 5-methylcytosine (5-mC) with a methyl group attached to the fifth carbon of a cytosine ring. The methylation of 5-methylcytosine occurs only at C of CG dinucleotide (5'-mCG-3') called CpG, and the methylation of CpG inhibits the expression of alu or transposon and a repeating sequence of a genome. In addition, since the 5-mC of the CpG is naturally deaminated to be easily thymine (T), the CpG is a site where most epigenetic changes frequently occur in mammalian cells.

In the present invention, the term "measurement of the methylation level" refers to measuring the methylation level of a CpG site of at least gene selected from the group consisting of IFFO1, MARCH11, BARHL2, NR2E1, KCNA3 and BOLL, and may be performed by a detection method according to the treatment of bisulfite or a bisulfite-free detection method. The methylation level may be measured by methylation-specific PCR, for example, methylation-specific polymerase chain reaction (MSP), real time methylation-specific polymerase chain reaction (PCR), PCR using a methylated DNA-specific binding protein, or quantitative PCR. Alternatively, the methylation level may be measured by automatic sequencing such as pyrosequencing and bisulfite sequencing, but is not limited thereto. In addition, the methylation level may be measured using a detection method using a ten-eleven translocation protein (TET protein) as the bisulfite-free detection method (see Nature Biotechnology, volume 37, pages 424-429 (2019)). The TET protein is an enzyme that acts on DNA and is involved in chemical changes of bases, and when bisulfite is treated, all Cs except for methylated C are converted to T bases, whereas in the Tet protein, only the methylated C is converted to T to enable effective detection.

Preferably, the CpG site of at least one gene selected from the group consisting of IFFO1, MARCH11, BARHL2, NR2E1, KCNA3 and BOLL refers to a CpG site present on DNA of the gene. The DNA of the gene is a concept including all of a series of structural units required for expression of the gene and operably linked to each other, and may include, for example, a promoter region, an open reading frame (ORF) and a terminator region. Accordingly, the CpG site of at least one gene selected from the group consisting of IFFO1, MARCH11, BARHL2, NR2E1, KCNA3 and BOLL may exist in a promoter region, an open reading frame (ORF) and a terminator region of the corresponding gene.

Preferably, in the present invention, the measuring of the methylation level of the CpG site of at least one gene selected from the group consisting of IFFO1, MARCH11, BARHL2, NR2E1, KCNA3 and BOLL may mean measuring the methylation level of cytosines in CpG sites of genes shown in Table 1 below.

**[Table 1]**

| Symbol | Genome Build | Chromosome | Region |
|---|---|---|---|
| IFFO1 | GRCh37 | 12 | 6664426-6665336 |
| MARCH11 | GRCh37 | 5 | 16179065-16180420 |
| BARHL2 | GRCh37 | 1 | 91185157-91185577 |
| NR2E1 | GRCh37 | 6 | 108485672-108490539 |
| KCNA3 | GRCh37 | 1 | 111216245-111217937 |
| BOLL | GRCh37 | 2 | 198649752-198651599 |

In the present invention, it is characterized that the CpG site of at least one gene selected from the group consisting of IFFO1, MARCH11, BARHL2, NR2E1, KCNA3 and BOLL is located between +/- 3000 bases (3 kb) from a transcription start site (TSS) of the gene.

In the present invention, a base sequence of a human genome chromosomal region was expressed according to The February 2009 Human reference sequence (GRCh37), but the expression of a specific sequence of the human genome chromosomal region may be slightly changed as the study results on the genomic sequence are updated, and the expression of the human genome chromosomal region of the present invention may vary according to the change. Accordingly, in the human genome chromosomal region expressed according to The February 2009 Human reference sequence (GRCh 37) of the present invention, since a human reference sequence has been updated after the filing date of the present invention, even if the expression of the human genome chromosomal region is changed differently from now, it will be apparent that the scope of the present invention affects the changed human genome chromosomal region. These changes may be easily seen by those with ordinary skill in the art to which the present invention pertains.

In the present invention, the agent for measuring the methylation level of the CpG site may include a compound of modifying a cytosine base or a methylation-sensitive restriction enzyme, a primer specific to a methylated allele sequence of at least one gene selected from the group consisting of IFFO1, MARCH11, BARHL2, NR2E1, KCNA3 and BOLL, and a primer specific to an unmethylated allele sequence.

The compound of modifying the cytosine base is a compound of modifying unmethylated cytosine or methylated cytosine, and may be bisulfite or a salt thereof, preferably sodium bisulfite of modifying the unmethylated cytosine, or a TET protein of modifying the methylated cytosine, but is not limited thereto. Methods for detecting whether the CpG site is methylated by modifying the cytosine base are well known in the art (WO01/26536; US2003/0148326A1).

In addition, the methylation-sensitive restriction enzyme may be a restriction enzyme capable of specifically detecting the methylation of the CpG site, and may be a restriction enzyme containing CG as a recognition site of the restriction enzyme. Examples thereof include SmaI, SacII, EagI, HpaII, MspI, BssHII, BstUI, NotI, and the like, but are not limited thereto. Depending on methylation or unmethylation at C of the restriction enzyme recognition site, cleavage by the restriction enzyme varies and may be detected through PCR or southern blot analysis. The methylation-sensitive restriction enzymes other than the restriction enzymes are well known in the art.

The primer may include a primer specific to a methylated allele sequence and a primer specific to an unmethylated allele sequence of at least one gene selected from the group consisting of IFFO1, MARCH11, BARHL2, NR2E1, KCNA3 and BOLL.

In the present invention, the term "primer" refers to a short nucleic acid sequence capable of forming a base pair with a complementary template and serving as a starting point for copying a template strand, as a nucleic acid sequence having a short free 3-terminal hydroxyl group. The primer may initiate DNA synthesis in the presence of a reagent for polymerization (i.e., DNA polymerase or reverse transcriptase) and four different nucleoside triphosphates in appropriate buffer and temperature. In addition, the primer is sense and antisense nucleic acids with a sequence of 7 to 50 nucleotides and may incorporate an additional feature without changing the basic properties of the primer that serves as an initiation site of DNA synthesis.

The primer of the present invention may be preferably designed according to a sequence of a specific CpG site to analyze the methylation, and more preferably, may be at least one selected the group consisting of a primer pair capable of specifically amplifying cytosine that is methylated to be unmodified by bisulfite, a primer pair capable of specifically amplifying cytosine that is not methylated to be modified by bisulfite, and a primer pair capable of specifically amplifying cytosine that is methylated to be modified by a Tet-based protein, and a primer pair capable of specifically amplifying cytosine that is not methylated and has not been modified by a Tet-based protein.

Accordingly, the present invention provides a kit for diagnosing bladder cancer comprising a primer pair for amplifying fragments including a CpG site of at least one gene selected from the group consisting of IFFO1, MARCH11, BARHL2, NR2E1, KCNA3 and BOLL.

In the composition and the kit, in addition to the preparation, polymerase agarose, a buffer solution required for electrophoresis, and the like may be additionally included.

Further, the present invention provides a nucleic acid chip for diagnosing bladder cancer in which a probe capable of hybridizing with fragments including a CpG site of at least one gene selected from the group consisting of IFFO1, MARCH11, BARHL2, NR2E1, KCNA3 and BOLL is immobilized.

In the present invention, the term "nucleic acid" refers to oligonucleotides, nucleotides, polynucleotides or fragments thereof, single- or double-stranded genomic or synthetic-origin DNA or RNA, sense or antisense-stranded genomic or synthetic-origin DNA or RNA, and peptide nucleic acid (PNA) or natural or synthetic-origin DNA or RNA analog material. If the nucleic acid is RNA, it will be apparent to those skilled in the art that instead of deoxynucleotides A, G, C and T, ribonucleotides A, G, C and U are substituted, respectively.

Since the methylation starts from the outside of a regulatory site of the gene and proceeds to the inside thereof, the gene involved in cell transformation may be diagnosed early and monitored for progression or recurrence after treatment by detecting the methylation outside the regulatory site.

Accordingly, it is possible to early diagnose cells likely to form bladder cancer using the methylated gene marker. When a gene confirmed to be methylated in cancer cells is methylated in clinically or morphologically normal-appearing cells, the normal-appearing cells are being cancerizated. Therefore, by confirming the methylation of a bladder cancer-specific gene in normal-appearing cells, it is possible to diagnose bladder cancer early and monitor the progression or recurrence after treatment thereof.

Further, the present invention provides a method for providing information for diagnosing bladder cancer comprising measuring the methylation level of a CpG site of at least one gene selected from the group consisting of IFFO1, MARCH11, BARHL2, NR2E1, KCNA3 and BOLL from a sample of a patient suspected of having bladder cancer; and comparing the measured methylation level with the methylation level of a CpG site of the same gene in a normal control sample.

The method for measuring the methylation level may be selected from the group consisting of PCR, methylation specific PCR, real time methylation specific PCR, PCR using a methylated DNA-specific binding protein, measuring whether there is methylation using a methylation-sensitive restriction enzyme, quantitative PCR, DNA chip, pyrosequencing and bisulfite sequencing, but is not limited thereto.

Specifically, the method of methylation-specific PCR is a method of designing and using different types of primers depending on whether CpG dinucleotide is methylated as a primer to perform PCR after treating a sample DNA with bisulfite. If the primer binding site has been methylated, PCR proceeds with the methylated primer, and if not methylated, PCR proceeds with a normal primer. That is, the method is a method of treating the sample DNA with bisulfite, performing PCR using two types of primers at the same time, and comparing the results.

The real time methylation specific PCR is to convert the methylation-specific PCR method to a real-time measurement method and to perform real time PCR by treating genomic DNA with bisulfite, designing PCR primers corresponding to methylation, and using these primers. At this time, there are two methods: a detection method using a TanMan probe complementary to an amplified base sequence and a detection method using Sybergreen. Accordingly, the real time methylation specific PCR may selectively quantitative-analyze only methylated DNA. In this case, the method is a method of preparing a standard curve using an in vitro methylated DNA sample, and quantitatively analyzing the methylation level by amplifying a gene without a 5'-CpG-3' sequence in a base sequence together as a negative control for standization.

In the method for measuring the methylation using the methylation-sensitive restriction enzyme, the methylation-sensitive restriction enzyme uses CpG dinucleotide as an action site, and does not act as the enzyme when this site is methylated. Therefore, when the sample DNA is treated with the methylation-sensitive restriction enzyme and then amplified by PCR to include an enzyme target site, the restriction enzyme does not act in the case of the methylation, but is amplified by PCR, but the unmethylated normal site is cleaved by the restriction enzyme and not amplified by PCR, thereby measuring whether a specific DNA site is methylated.

In the PCR or DNA chip method using the methylated DNA-specific binding protein, when a protein that specifically binds only to methylated DNA is mixed with DNA, the protein specifically binds only to the methylated DNA, so that only the methylated DNA may be selectively isolated. After mixing genomic DNA with the methylated DNA-specific binding protein, only the methylated DNA is selectively isolated. This is a method of amplifying these isolated DNAs using a PCR primer corresponding to an intron site, and then measuring whether methylation occurs by agarose electrophoresis. In addition, the methylation may be measured even by quantitative PCR, and the methylated DNA isolated by the methylated DNA-specific binding protein is labeled with a fluorescent dye and hybridized to a DNA chip integrated with a complementary probe to measure the methylation. Here, the methylated DNA-specific binding protein is not limited to MBD2bt.

In addition, pyrosequencing of bisulfite-treated DNA is based on the following principle. When methylation occurs at a CpG dinucleotide site, 5-methylcytosine (5-mC) is formed, wherein the modified base is changed to uracil upon treatment with bisulfite. If the CpG dinucleotide has been methylated when the DNA extracted from the sample is treated with bisulfite, the CpG dinucleotide is preserved as cytosine and the remaining unmethylated cytosines are changed to uracils. Sequencing of the bisulfite-treated DNA may be preferably performed using a pyrosequencing method. Detailed descriptions of pyrosequencing are known in the art.

Meanwhile, by the bisulfite-free detection method using the Tet protein, only methylated C may be converted to T using the Tet protein to detect the base at the methylated region (see LIU, Yibin, et al., Nature Biotechnology volume 37, pages 424-429 (2019)).

When the methylation occurs at the CpG dinucleotide site so that cytosine is formed as 5-methylcytosine (5-mC), the CpG dinucleotide has been methylated when treated with the ten-eleven translocation (Tet) protein to be changed to uracil, and unmethylated cytosines are preserved. The sequencing of Tet-treated DNA is not limited only to the pyrosequencing method, but may be performed by using methods such as methylation-sensitive PCR (MSP), microarray, next generation sequencing (NGS), and the like.

Preferably, the method for providing information for diagnosing bladder cancer of the present invention may be performed by the method comprising the steps of a) obtaining a sample from a subject, b) obtaining genomic DNA from the sample, c) treating the obtained genomic DNA with a compound that modifies unmethylated cytosine bases, d) obtaining a PCR product by amplifying the treated DNA by PCR using a primer for pyrosequencing capable of amplifying a promoter of at least one selected from the group consisting of IFFO1, MARCH11, BARHL2, NR2E1, KCNA3 and BOLL, and e) measuring the methylation level by pyrosequencing the PCR product using a sequencing primer.

The obtaining of the genomic DNA in step b) may be performed using a phenol/chloroform extraction method, an SDS extraction method, a CTAB separation method, or a commercially available DNA extraction kit, which is commonly used in the art.

In the present invention, the term "sample" refers to a wide range of bodily fluids, including all biological fluids obtained from individuals, bodily fluids, cell lines, tissue cultures, etc., depending on a type of analysis to be performed. Methods for obtaining bodily fluids and tissue biopsies from mammals are generally widely known, and in the present invention, the samples may be preferably selected from the group consisting of human derivatives including tissues, cells, blood, plasma, serum, feces, and urine. Since abnormal methylation changes in cancer tissues show significant similarities to methylation changes in genomic DNA obtained from a biological sample such as cells, whole blood, serum, plasma, saliva, sputum, cerebrospinal fluid, or urine, in the case of using the marker of the present invention, with respect to the prediction of the occurrence of bladder cancer, there is an advantage that it is possible to be easily diagnosed through blood, bodily fluids, or the like.

Meanwhile, the present invention provides a method for measuring the methylation level of a CpG site of at least one gene selected from the group consisting of IFFO1, MARCH11, BARHL2, NR2E1, KCNA3 and BOLL in a biological sample isolated from a subject, in order to provide information required for diagnosing the recurrence or progression of bladder cancer.

In the present method, the "subject" may be animals, preferably animals including mammals, particularly humans, and may be cells, tissues, organs, etc. derived from animals. The subject may be a patient in need of the effect, preferably a patient suspected of bladder cancer, a patient diagnosed with bladder cancer, or a patient receiving treatment for bladder cancer.

In addition, the "sample" is the same as described above, but preferably, the sample for diagnosing reoccurrence or progression may be urine obtained from a subject or patient.

In the present invention, the reoccurrence or progression diagnosis of bladder cancer may include the prognosis of bladder cancer. The "prognosis" refers to the course of disease progression during or after treatment for bladder cancer, and preferably refers to the course of disease progression after treatment, and includes overall survival, disease free survival or distant metastasis free survival, but is not limited thereto. In addition, the progression of bladder cancer is a concept including cure, recurrence, metastasis or metastatic recurrence of bladder cancer, and more preferably means metastatic recurrence, but is not limited thereto.

The present invention provides the use of an agent for measuring the methylation level of a CpG site of at least one gene selected from the group consisting of IFFO1, MARCH11, BARHL2, NR2E1, KCNA3 and BOLL for preparing an agent for diagnosing bladder cancer.

The present invention provides a method for diagnosing bladder cancer comprising the steps of:
a) obtaining a sample from a subject;
b) measuring the methylation level of a CpG site of at least one gene selected from the group consisting of IFFO1, MARCH11, BARHL2, NR2E1, KCNA3 and BOLL from the sample; and
c) comparing the measured methylation level with the methylation level of a CpG site of the same gene in a normal control sample.

In an aspect, the present invention provides a method for diagnosing and treating bladder cancer of a subject comprising the following steps:
i) obtaining a sample from a subject;
ii) measuring the methylation level of a CpG site of at least one gene selected from the group consisting of IFFO1, MARCH11, BARHL2, NR2E1, KCNA3 and BOLL from the sample;
iii) comparing the measured methylation level with the methylation level of a CpG site of the same gene in a normal control sample; and
iv) treating the bladder cancer by administering a therapeutic drug for treating the bladder cancer to the determined subject or through surgery.

Methods including steps i) to iv) are understood based on the method including steps a) to c) described above.

Step iv) is a step of performing the treatment of the diseases by a means such as administration of the therapeutic drug or surgery, to the subject in which the disease is diagnosed in step iii).

The 'treatment' of the present invention comprehensively refers to improving bladder cancer or symptoms thereof, and may include treating or substantially preventing the disease, or improving the conditions thereof and includes alleviating, treating or preventing a symptom or most of symptoms derived from bladder cancer, but is not limited thereto.

The type of 'therapeutic drug' is not particularly limited as long as the therapeutic drug is any type of drug typically used for the treatment of bladder cancer. In addition, the therapeutic drug is administered to a subject in a 'therapeutically effective dose', wherein the therapeutically effective dose for patients may be determined by those skilled in the art by considering various factors, such as age, weight, health condition, and sex of a patient, severity of a disease, diet and excretion rate, etc. as well as unique properties, route of administration, and treatment times of the drug. The route of administration of the therapeutic drug is not particularly limited, and the therapeutic drug may be administered orally or parenterally, and includes both local administration and systemic administration. The parenteral administration is not limited thereto, but may be, for example, intranasal drug application, subcutaneous injection, and the like, and as another example, a method such as intramuscular injection, intravenous injection, or the like may be used.

The 'sample' of the present invention is isolated and obtained from a subject suspected of having a disease, but is not limited thereto, but may be selected from the group consisting of cells, tissues, blood, serum, plasma, saliva, sputum, mucosal fluid, and urine. The 'subject' may be animals, preferably animals including mammals, particularly humans, and may be cells, tissues, organs, etc. derived from animals. The subject may be a patient requiring the therapeutic effects.

The term "comprising" used herein is used in the same meaning as "including" or "characterized by", and does not exclude additional ingredients or steps of the method which are not specifically mentioned in the composition or the method according to the present invention. The term "consisting of' means excluding additional elements, steps or ingredients, etc., unless otherwise described. The term "essentially consisting of' means including materials or steps which do not substantially affect basic properties thereof in addition to the described materials or steps within the range of the composition or the method.

### ADVANTAGEOUS EFFECTS

As described above, since hypermethylation of the CpG site of at least one selected from the group consisting of IFFO1, MARCH11, BARHL2, NR2E1, KCNA3 and BOLL is specifically exhibited in bladder cancer, it is possible to accurately and quickly diagnose bladder cancer, and also to monitor for early diagnosis and recurrence after treatment using a composition, a kit, a chip or a method according to the present invention.

### DESCRIPTION OF DRAWINGS

FIG. 1 is a result of confirming a difference in methylation between a tumor tissue (tumor) group and a non-tumor tissue (normal) group of a total of six genes selected according to the present invention.
FIG. 2 is a result confirming methylation information of a total of six genes selected according to the present invention in a tumor tissue (tumor) cell line group.
FIG. 3 is a result of confirming methylation information of a total of six genes selected according to the present invention in peripheral blood mononuclear cells.
FIG. 4 is a result of confirming methylation information of a total of six genes selected according to the present invention for each type of peripheral blood mononuclear cells.
FIG. 5 is a result of confirming the diagnosis accuracy of bladder cancer of a total of 6 genes selected according to the present invention.

### MODES FOR THE INVENTION

Hereinafter, preferred Examples will be proposed in order to help in understanding of the present invention. However, the following Examples are just provided to more easily understand the present invention and the contents of the present invention are not limited by Examples.

### Example 1: Selection of bladder cancer-specific methylated gene

To select a methylated gene specifically found in bladder cancer, a comparative study on large-scale methylation of cancer and normal tissues obtained from cancer surgery in bladder cancer patients was performed using large-scale methylation microarray chip data. The tumor tissue used in this study means cancer tissue of bladder cancer, and the non-tumor tissue means tissues including normal tissue other than the cancer tissue. The number of bladder cancer tissues used in the analysis was 412 and the number of non-tumor tissues was 21.

In order to select bladder cancer-specific methylated genes, DNA was extracted from each tissue, and the methylation level of a gene region was confirmed using an Infinium Human Methylation 450 Beadchip microarray.

DNA extracted from each tissue was converted through bisulfite treatment, and as a result, a cytosine base was modified depending on whether a DNA site was methylated. A probe used in the corresponding microarray experiment was specifically designed for methylation and unmethylation in order to confirm whether a cytosine base in a methylated region of a gene was modified.

The microarray experiment measured the methylation level of the gene through about 450,000 (450 k) probes representing the methylated region of each gene, and a result of each probe derived from the experiment was presented as a beta value. The beta value had a value of 0 to 1, and it was determined that the closer to 1, the higher the methylation level of the corresponding gene region.

In order to identify differentially methylated regions (DMRs) between the tumor group and the non-tumor group, by using an empirical Bayes t-test, a Linear Models for Microarray Data (Limma) method, a gene region having a statistically significant methylation difference between the groups was identified.

The Limma method has been known to be least affected by an outlier among several methylation statistical analysis methods to identify the difference between the groups. Therefore, the method was a suitable method for finding cancer-specific markers because the method was less affected by abnormal measurement values of some samples. In the present experiment, it was determined that there was a significant difference in methylation between the two groups as an adjusted p-value derived through the Limma method was smaller.

In particular, in order to search for tumor-specific methylated regions, among gene regions with a significant difference in beta values between tumor and non-tumor groups, gene regions with higher methylation in tumor tissue than in non-tumor tissue were selected as cancer-specific biomarker candidates.

As a result, as a result of limma analysis in a dataset, compared with the non-tumor group, when comparing the tumor groups, gene regions having a low p value within the 10,000-th among 450,000 probes, and a large difference in beta value of 0.3 or more between the groups were selected as tumor-specific hypermethylated regions. As a result, among about 450,000 gene regions, 352 gene regions exhibiting tumor-specific hypermethylation in the dataset were selected as biomarker candidates.

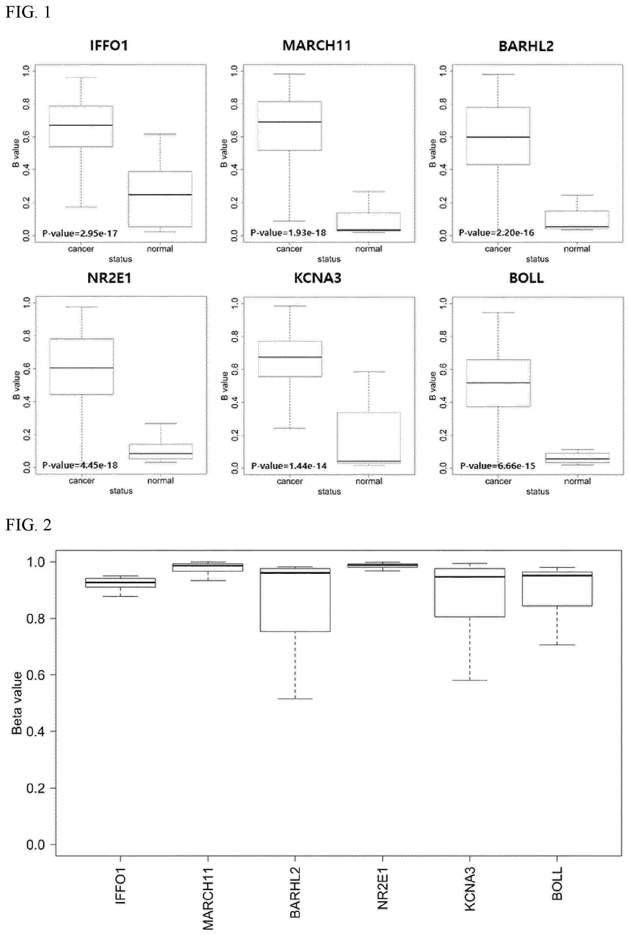

Among these gene regions, when the corresponding region was not a pseudogene, existed in a CpG island site, was located in a gene region selected between +/- 3000 bases (3 kb) from a transcription start site (TSS) of the gene, and existed in an autosome, the corresponding region was selected as a bladder cancer-specific hypermethylated gene. As a result, as shown in Table 2 below, a total of 6 genes were selected (see FIG. 1).

**[Table 2]**

| **Symbol** | **Name** | **Location (Chromosome)** | **CpG Island** |
|---|---|---|---|
| IFFO1 | intermediate filament family orphan 1 (IFFO; HOM-TES-103) | 12 | Island |
| MARCH11 | membrane associated ring-CH-type finger 11 (RNF226; MARCHF 11; MARCH-XI) | 5 | Island |
| BARHL2 | BarH like homeobox 2 | 1 | Island |
| NR2E1 | nuclear receptor subfamily 2 group E member 1 | 6 | Island |
| KCNA3 | potassium voltage-gated channel subfamily A member 3 (MK3; HGK5; HLK3; PCN3; HPCN3; KV1.3; HUKIII) | 1 | Island |
| BOLL | boule homolog, RNA binding protein (BOULE) | 2 | Island |

### Example 2: Confirmation of bladder cancer specificity of bladder cancer diagnostic gene in cell line

In order to confirm whether the high methylation of the six selected genes was caused by bladder cancer cells, methylated patterns in 20 cancer cell lines derived from bladder cancer were largely analyzed using a public database. For the corresponding data, the Infinium Human Methylation 450 Beadchip microarray experiment was performed on DNA extracted from each cell line according to a manufacturer's standardized methylation analysis test procedure.

In the result values of the performed experiment, the methylation levels of genes were measured through about 450,000 probes as in Example 1, and the methylation value of each probe was presented as a beta value. The beta value had a value of 0 to 1, and it was determined that the closer to 1, the higher the methylation level of the corresponding gene region.

The 20 bladder cancer cell lines were as follows: 5637 (comic ID: 687452), 639-V (comic ID: 906798), 647-V (comic ID: 906797), BFTC-905 (comic ID: 910926), CAL-29 (comic ID: 1290730), DSH1 (comic ID: 753552), HT-1197 (comic ID: 907065), HT-1376 (comic ID: 907066), J82 (comic ID: 753566), KU-19-19 (comic ID: 907312), LB831-BLC (comic ID: 753584), RT-112 (comic ID: 909704), RT4 (comic ID: 687455), SCABER (comic ID: 1299051), SW1710 (comic ID: 909749), SW780 (comic ID: 687457), T-24 (comic ID: 724812), TCCSUP (comic ID: 687459), UM-UC-3 (comic ID: 724838), VM-CUB-1 (comic ID: 909780)

As a result of confirming the methylation level in the bladder cancer cell line of the six selected genes, a median methylation value of each gene was 0.93 or higher, which showed a high level of methylation value as in the high methylation level of the selected gene in the tumor tissue (see FIG. 2). Therefore, it was confirmed that the selected gene had specific methylation in bladder cancer.

### Example 3: Confirmation of low methylation level of bladder cancer diagnostic gene in peripheral blood mononuclear cells

Peripheral blood mononuclear cells (PBMC) were peripheral blood cell with a spherical nucleus. These peripheral blood mononuclear cells include immune-related cells such as T cells, B cells, macrophages, dendritic cells, natural killer cells (NK cells, natural killer cells), and the like.

In order to confirm the methylation levels of the selected six genes, a step of obtaining cancer samples was required. These samples refer to a wide range of body fluids, including all cancer-related biological fluids obtained from individuals, bodily fluids, cell lines, tissue cultures, etc., depending on a type of analysis to be performed. In the process of obtaining the samples, contamination of peripheral blood mononuclear cells was a cause for inhibiting measurement of methylation occurring most frequently, and affected the gene methylation levels in cancer samples by methylation of genes to be measured in peripheral blood mononuclear cells.

In order to determine whether the selected six genes were affected by the contamination of the peripheral blood mononuclear cells, methylated patterns of the peripheral blood mononuclear cells were analyzed from a total of 110 subjects. For the data, DNA extracted from the peripheral blood mononuclear cells was subjected to an Infinium Human Methylation 450 Beadchip microarray experiment according to a standardized manufacturer's methylation analysis test process as in Examples 1 and 2. In the result values of the performed experiment, the methylation levels of genes were measured through about 450,000 probes, and the methylation value of each probe was presented as a beta value. The beta value had a value of 0 to 1, and it was determined that the closer to 1, the higher the methylation level of the corresponding gene region.

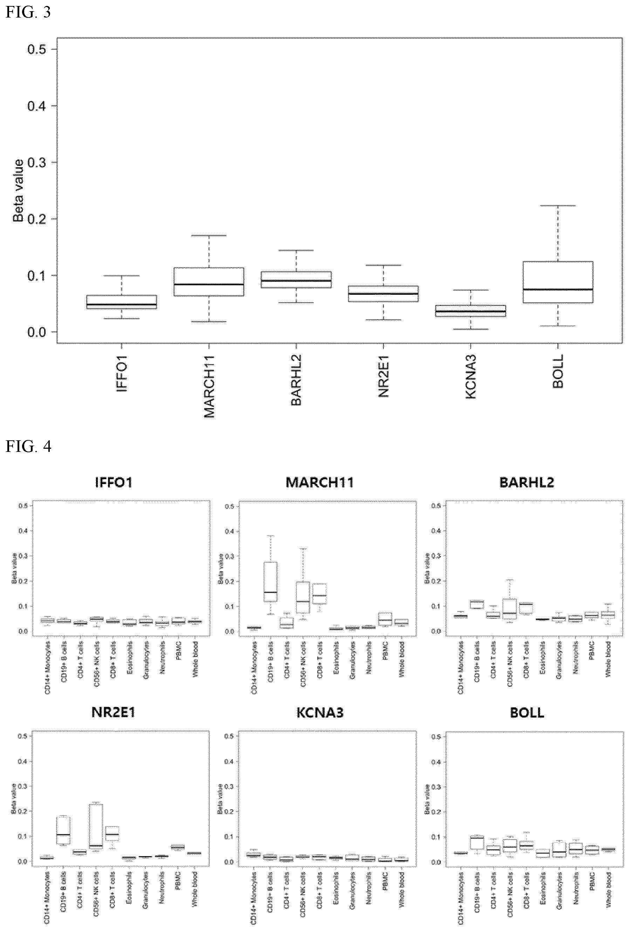

As a result of confirming the methylation levels in the peripheral blood mononuclear cells of the six selected genes, a median methylation value of each gene was less than 0.10, which showed a low level of methylation value as in the high methylation level of the selected gene in the tumor tissue (see FIG. 3). Therefore, it was confirmed that the selected gene had specific methylation in bladder cancer.

In addition, methylation analysis was performed by cell group constituting the peripheral blood mononuclear cells to analyze whether a specific cell group affected the measurement of the methylation level in the sample. In a total of six normal blood samples, the cell groups were divided into whole blood, peripheral blood mononuclear cells (PBMC), and granulocytes, CD4+ T cells, CD8+ T cells, CD56+ NK cells, CD19+ B cells, CD14+ cells, monocytes, neutrophils, and eosinophils constituting the PBMCs to analyze the DNA methylation levels. The methylation level was measured through the Infinium Human Methylation 450 Beadchip microarray experiment as in Examples 1 and 2.

As a result of confirming the levels for each sub-cell group of peripheral blood mononuclear cells of the six selected genes, a median methylation value of each gene was at most less than 0.16, and the selected genes also showed low methylation level values even in each of the peripheral blood mononuclear cells (see FIG. 4).

These results mean that since the selected genes exhibited low methylation levels in the peripheral blood mononuclear cells, the effect from the contamination of the peripheral blood mononuclear cells on the measurement of methylation of the corresponding gene from the sample is extremely limited.

### Example 4: Evaluation of diagnostic performance of bladder cancer diagnostic genes

In order to confirm the usefulness of the selected genes as a diagnostic marker in bladder cancer, the accuracy of diagnosis of bladder cancer according to the methylation level was evaluated.

In order to evaluate the accuracy of diagnosis, sensitivity and specificity were used. A receiver operating characteristic (ROC) curve that presented changes in sensitivity and specificity according to a cut-off value may be shown through the calculation of the sensitivity and specificity values for feasible cut-off values of consecutive diagnostic test measurement values. The accuracy of diagnosis may be measured by an area under the ROC curve (AUC). The AUC value has a value between 0.5 and 1, and it is evaluated that the higher the value, the higher the diagnostic accuracy. If the AUC value is 1, it is meant that the diagnostic result is a perfectly accurate test, but if the AUC value is 0.5, it is determined that the diagnostic result is the same as the random result.

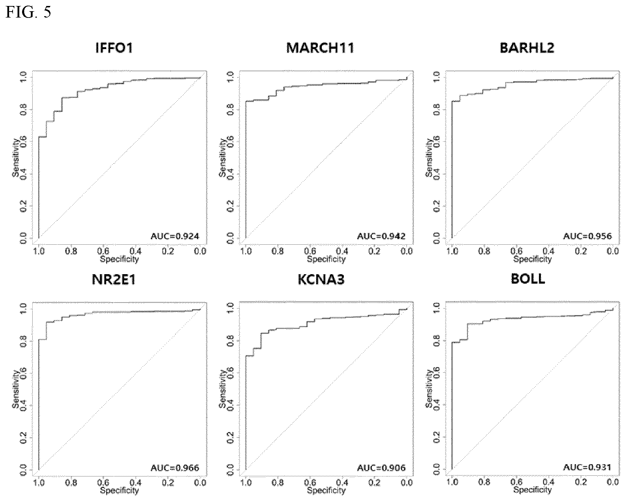

As a result of analyzing the cancer classification accuracy according to the methylation level between the non-tumor tissue and the tumor tissue using the selected genes by using a collected methylation dataset, as illustrated in FIG. 7, it was confirmed that all the selected genes had area under curve (AUC) values of 0.906 or higher to have high diagnostic accuracy, so that the selected genes were useful for diagnosing bladder cancer (see FIG. 5).

### INDUSTRIAL APPLICABILITY

As described above, since hypermethylation of the CpG site of at least one selected from the group consisting of IFFO1, MARCH11, BARHL2, NR2E1, KCNA3 and BOLL is specifically exhibited in bladder cancer, it is possible to accurately and quickly diagnose bladder cancer, and also to monitor for early diagnosis and recurrence after treatment using a composition, a kit, a chip or a method according to the present invention.

## Claims

1. A composition for diagnosing bladder cancer comprising an agent for measuring the methylation level of a CpG site of at least one gene selected from the group consisting of IFFO1, MARCH11, BARHL2, NR2E1, KCNA3 and BOLL.

2. The composition of claim 1, wherein the CpG site is located between +/- 3000 bases (3 kb) from a transcription start site of the gene.

3. The composition of claim 1, wherein the agent for measuring the methylation level of the CpG site of the gene is selected from the group consisting of:
a compound of modifying an unmethylated cytosine or methylated cytosine base;
a primer specific to a methylated sequence of a CpG site of at least one gene selected from the group consisting of IFFO1, MARCH11, BARHL2, NR2E1, KCNA3 and BOLL from the sample; and
a primer specific to the unmethylated sequence.

4. The composition of claim 3, wherein the compound of modifying the unmethylated cytosine base is bisulfite or a salt thereof and the compound of modifying the methylated cytosine base is a Tet protein.

5. A kit for diagnosing bladder cancer comprising a primer pair for amplifying fragments including a CpG site of at least one gene selected from the group consisting of IFFO1, MARCH11, BARHL2, NR2E1, KCNA3 and BOLL.

6. A nucleic acid chip for diagnosing bladder cancer in which a probe capable of hybridizing with fragments including a CpG site of at least one gene selected from the group consisting of IFFO1, MARCH11, BARHL2, NR2E1, KCNA3 and BOLL is immobilized.

7. A method for providing information for diagnosing bladder cancer comprising:
measuring the methylation level of a CpG site of at least one gene selected from the group consisting of IFFO1, MARCH11, BARHL2, NR2E1, KCNA3 and BOLL from a sample of a patient suspected of having bladder cancer; and
comparing the measured methylation level with the methylation level of a CpG site of the same gene in a normal control sample.

8. The method of claim 7, wherein the method for measuring the methylation level is selected from the group consisting of a bisulfite-free detection method, methylation-specific polymerase chain reaction, real time methylation-specific polymerase chain reaction, PCR using a methylated DNA-specific binding protein, quantitative PCR, pyrosequencing, and bisulfite sequencing.

9. The method of claim 7, wherein the sample is selected from the group consisting of tissues, cells, blood, plasma, serum, feces, and urine.

10. A method for measuring the methylation level of a CpG site of at least one gene selected from the group consisting of IFFO1, MARCH11, BARHL2, NR2E1, KCNA3 and BOLL in a biological sample isolated from a subject, in order to provide information required for diagnosing the recurrence or progression of bladder cancer.

11. The method of claim 10, wherein the subject is a patient diagnosed with bladder cancer or a patient who has been treated for bladder cancer.

12. The method of claim 10, wherein the biological sample is urine.

13. Use of an agent for measuring the methylation level of a CpG site of at least one gene selected from the group consisting of IFFO1, MARCH11, BARHL2, NR2E1, KCNA3 and BOLL for preparing an agent
for diagnosing bladder cancer.

14. A method for diagnosing bladder cancer comprising the steps of:
a) obtaining a sample from a subject;
b) measuring the methylation level of a CpG site of at least one gene selected from the group consisting of IFFO1, MARCH11, BARHL2, NR2E1, KCNA3 and BOLL from the sample; and
c) comparing the measured methylation level with the methylation level of a CpG site of the same gene in a normal control sample.
